# EUROPÄISCHE PATENTSCHRIFT

(11) **EP 2 361 255 B1**
(45) Veröffentlichungstag und Bekanntmachung des Hinweises auf die Patenterteilung: **11.06.2014**
(21) Anmeldenummer: 10800717.0
(22) Anmeldetag: 15.12.2010
(51) Int. Cl.: C07H 1/00, C07H 15/04, A23L 1/236

(54) **SÜßUNGSMITTEL UND VERFAHREN ZU SEINER HERSTELLUNG**
SWEETENER AND METHOD FOR THE PRODUCTION THEREOF
ÉDULCORANT ET SON PROCÉDÉ DE PRÉPARATION

(30) Priorität: 23.12.2009 DE 102009055256
(43) Veröffentlichungstag der Anmeldung: 31.08.2011
(73) Patentinhaber: Evonik Degussa GmbH, 45128 Essen (DE)
(72) Erfinder: ZEHNACKER, Olivier, 45657 Recklingshausen (DE); TACKE, Thomas, 63755 Alzenau (DE); HAAS, Thomas, 48161 Münster (DE); BRAUSCH, Nicole, 45130 Essen (DE); BECKER, Marc, 44359 Dortmund (DE)
(86) Internationale Anmeldenummer: PCT/EP2010/069726
(87) Internationale Veröffentlichungsnummer: WO 2011/076625

(56) Entgegenhaltungen:
- EP-A1- 0 625 578
- EP-A1- 0 854 148
- DE-A1- 19 523 008

## Beschreibung

### Gebiet der Erfindung

Gegenstand der Erfindung ist ein Süßungsmittel so wie ein Verfahren zu seiner Herstellung.

### Stand der Technik

Isomalt (auch Isomaltit, Isomaltitol, Palatinit®) ist ein Zuckeraustauschstoff, der aus Saccharose gewonnen wird. Seine Herstellung erfolgt in einem Zweistufen-Prozess: Zunächst wird Saccharose durch Umlagerung in Isomaltulose (α-D-Glucopyranosyl-1,6-Fruktose, auch Palatinose®) überführt. Die aufgereinigte Isomaltulose wird anschließend durch katalytische Hydrierung zu Isomalt umgesetzt.
Im Rahmen der Hydrierung der Isomaltulose entstehen zwei Isomere, das α-D-Glucopyranosyl-1,1-D-Mannitol (im Folgenden als 1,1-GPM bezeichnet) und das α-D-Glucopyranosyl-1,6-D-Sorbitol (im Folgenden als 1,6-GPS bezeichnet), aus dem Isomalt im Wesentlichen besteht.

Die Isomerisierung der Saccharose zu Isomaltulose wird in der Regel enzymatisch mit Isomaltulose Synthasen (Saccharose Glucosylmutasen, EC 5.4.99.11) durchgeführt.
DE1049800, DE2217628, EP 28900, EP49472 und EP 91063 beschreiben Verfahren mit immobilisierten Bakterienzellen zur enzymatischen Umwandlung der Saccharose zu Isomaltulose. EP 0625578 setzt hierzu Bakterienstämme aus der Gruppe von *Protaminobacter rubrum* (CBS 574.77), *Serratia plymuthica* (ATCC 15928), Serratia marcescens (NCIB 8285), Leuconostoc mesenteroides (NRRL-B 512 F (ATCC 1083 a)) und *Erwinia rhapontici* (NCPPB 1578) ein. EP 0392556 und EP1257638 beschreiben den Einstaz von Bakterienstämmen aus der Gruppe von *Klebsiella terrigena* JCM 1687, *Klebsiella sp.* No. 88 (FERM BP-2838) und *Klebsiella singaporensis* LX3 and LX21.
Diese Isomerisationsverfahren werden sowohl mit lebenden oder toten Zellen, mit immobilisierten oder freien Zellen durchgeführt: so beschreiben DE3133123 und EP0915986 zum Beispiel Immobilisierungsverfahren der Enzymkatalysatoren mit Calciumalginat oder Ionentauschern, so wie die EP0001099 ein Verfahren mit freien, lebenden Zellen, die Isomaltulose im Rahmen einer Fermentation herstellen können.
Allen bekannten Verfahren zur Isomerisierung ist gemein, dass die Saccharose nie vollständig umgesetzt wird, somit immer in Spuren nachweisbar bleibt, und zur weiteren Verarbeitung der Isomaltulose zu Isomalt eine Abtrennung der nicht isomerisierten Saccharose durchgeführt werden muss.

Zur Abtrennung der nicht isomerisierten Saccharose wird in der Regel eine Kristallisation der Isomaltulose durchgeführt. Solche Verfahren werden beispielsweise in EP0091063 und EP1550666 beschrieben.
EP0625578 beschreibt ein Verfahren, bei dem die Entfernung der nicht isomerisierten Saccharose durch eine zusätzliche Spaltung in die korrespondierenden Monosaccharide Fruktose und Glucose und deren Abtrennung erreicht wird.

Die Hydrierung der Isomaltulose ist allgemein hin bekannt, und es werden in beispielsweise GB1429334, DE2520173 und EP0625578 Verfahren beschrieben, die Raney-Nickel, als Katalysatoren bei erhöhten Drücken und Temperaturen verwenden.

Des Weiteren sind aus EP152779 und DE-A 4416115 Verfahren zur kontinuierlichen Hydrierung der Isomaltulose bekannt, die trägerfreie Formkörper von Elementen der 8. Nebengruppe des Periodensystems bzw. trägerfreie Formkörper von Elementen der Eisenuntergruppe der 8. Nebengruppe des Periodensystem mit Elementen der 6. Nebengruppe als Katalysator einsetzen.
Die EP0854148 beschreibt ein Verfahren zu Hydrierung von Isomaltulose an einem Katalysator enthaltend Nickel, Nickeloxid und Wolframoxid.
EP0838468 beschreibt ein Verfahren zur Hydrierung von Isomaltulose an als Hydrierungskatalysatoren dienenden trägerfreien Formkörpern enthaltend Legierungen von Elementen der Eisenuntergruppe der VIII. Nebengruppe des Periodensystem mit Elementen der IV. und/oder V. Nebengruppe des Periodensystems.
DE19523008 beschreibt ein Verfahren zur Hydrierung von Isomaltulose, um bestimmte Verhältnisse von 1,1-GPM zu 1,6-GPS zu erreichen unter Verwendung eines Katalysators aus Ruthenium, Nickel und deren Mischungen auf einem inerten Träger
Die DE19523008 beschreibt ein Verfahren zu Hydrierung von Isomaltulose an einem Katalysator enthaltend Ruthenium und/oder Nickel auf einem inerten Träger zur Steuerung des IsomerenVerhältnisses.

Da bei der enzymatischen Umsetzung der Saccharose als Nebenprodukt oft Trehalulose (α-D-Glucopyranosyl-1,1-Fruktose) sowie Fruktose und Glukose gebildet werden, kann diese je nach durchgeführter Reinigung nach der Isomerisierungsstufe in die Hydrierungsreaktion gelangen. Trehalulose wird dort zu α-D-Glucopyranosyl-1,1-D-Mannitol und zu α-D-Glucopyranosyl-1,1-D-Sorbitol (im Folgenden als 1,1-GPS bezeichnet) sowie Fruktose und Glukose zu Sorbitol und Mannitol umgesetzt.
Somit kann Isomalt zu Weilen neben den wesentlichen Bestandteilen 1,1-GPM und 1,6-GPS auch 1,1-GPS, Mannitol und Sorbitol enthalten.
Solch Isomalt und Verfahren zu seiner Herstellung werden beispielsweise in der JP -A 751079 und der EP0625578 beschrieben.

Einer der Hauptnachteile aller bekannten Verfahren zur Herstellung von auf Saccharose basierenden diätetischen Süßungsmitteln wie Isomalt ist die Notwendigkeit der Abtrennung der stark glykämisch wirkenden Rest-Saccharose nach der enzymatischen Isomerisierung des Ausgangszuckers. EP0625578 beschreibt diese verbleibende Rest-Saccharose daher treffend als explizit "nicht hydrierfähig".
Bei der Abtrennung der Rest-Saccharose durch oben beschriebene Verfahren kommt es zwangsläufig zu Verlusten an Isomaltulose oder anderen wertvollen Produkten.

Die Aufgabe der vorliegenden Erfindung besteht darin, ein auf Saccharose basiertes Süßungsmittel bereitzustellen, bei dessen Herstellung der Abtrennungsschritt der Rest-Saccharose aus der Isomerisierungsstufe entfällt und welches über hervorragend Eigenschaften zur weiteren Verarbeitung wie beispielsweise Formulierbarkeit in Süßigkeiten aufweist.

### Beschreibung der Erfindung

Überraschenderweise wurde gefunden, dass das in Anspruch 1 beschriebene Süßungsmittel so wie das im Folgenden beschriebene Verfahren zu seiner Herstellung einen Beitrag zur Lösung der eingangs genannten Aufgabe leistet.

Gegenstand der vorliegenden Erfindung sind daher ein auf Saccharose als Ausgangsstoff basierendem Süßungsmittel.
Ein weiterer Gegenstand der Erfindung ist ein katalytisches Verfahren, welches die gleichzeitige Hydrierung von Isomaltulose und gegebenenfalls Trehalulose in Isomalt und Saccharose in Sorbitol und Mannitol erlaubt.

Ein Vorteil des erfindungsgemäßen Süßungsmittels ist, dass es im Vergleich zum herkömmlichen Isomalt und bezogen auf 1,1-GPM angereichert an 1,6-GPS ist, welches eine starke Süßkraft und ein gutes Lösungsverhalten in Wasser aufweist; dies ist ebenfalls der Vorteil des erfindungsgemäßen Verfahrens, da dieses direkt solch ein Süßungsmittel als Produkt zur Verfügung stellt.
Ein Vorteil des erfindungsgemäßen Verfahrens ist es außerdem, dass dieses bei relativ niedrigen Temperaturen und Drücken durchgeführt werden kann und somit energie- und ressourcenschonend ist.

Unter dem Begriff "Rest-Saccharose" wird im Zusammenhang mit der vorliegenden Erfindung der Saccharoseanteil verstanden, der bei der Umsetzung der eingangs eingesetzten Saccharose mit einer Saccharose Mutase nicht umgesetzt worden ist und als Saccharose neben der zu beispielsweise Isomaltulose oder Trehalulose isomerisierten Saccharose vorliegt.

Unter dem Begriff "Süßungsmittel" wird im Zusammenhang mit der vorliegenden Erfindung ein Gemisch von Verbindungen verstanden, welches in flüssiger oder fester Form, kristallin oder gelöst vorliegen kann, gegebenenfalls Wasser enthalten kann und süß schmeckt.

Unter dem Begriff "saurer Träger" wird im Zusammenhang mit der vorliegenden Erfindung dem Fachmann als ein "saurer Träger" geläufiger Träger, wie beispielsweise Metalloxide, wie Al₂O₃, SiO₂, TeO₂ oder Mischoxide derselben, bezeichnet, der durch seine intrinsische Eigenschaften eine Acidität aufweist, aber auch solch ein Träger, der erst durch geeignete Behandlung saure Funktionalitäten auf der Oberfläche aufweist; dabei kann es sich beispielsweise um Trägermaterialien handeln, die mit Säuren wie z. B. Phosphorsäure behandelt werden oder aber um Träger, bei denen erst durch die Aufbringung der Aktivkomponente Ruthenium z. B. als Rutheniumchlorid in saurer Lösung eine saure Funktionalität eingeführt wird; solch ein saurer Träger ist beispielweise eine mit Rutheniumchlorid in saurer Lösung imprägnierte Aktivkohle.
Alle angegebenen Prozent (%) sind, wenn nicht anders angegeben, Massenprozent.

Einen Beitrag zur Lösung der vorstehend genannten Aufgaben leistet ein Süßungsmittel enthaltend, bevorzugt bestehend aus
20 Gew.-% bis 75 Gew.-%, bevorzugt 40 Gew.-% bis 60 Gew.-%, besonders bevorzugt 45 Gew.-% bis 57 Gew.-% α-D-Glucopyranosyl-1,6-D-Sorbitol,
20 Gew.-% bis 75 Gew.-%, bevorzugt 40 Gew.-% bis 60 Gew.-%, besonders bevorzugt 45 Gew.-% bis 55 Gew.-% α-D-Glucopyranosyl-1,1-D-Mannitol,
0,02 Gew.-% bis 15 Gew.-%, bevorzugt 0,1 Gew.-% bis 10 Gew.-%, besonders bevorzugt 0,2 Gew.-% bis 5 Gew.-% α-D-Glucopyranosyl-1,1-D-Sorbitol,
0,02 Gew.-% bis 15 Gew.-%, bevorzugt 0,1 Gew.-% bis 8 Gew.-%, besonders bevorzugt 0,2 Gew.-% bis 3,5 Gew.-% Sorbitol und 0,02 Gew.-% bis 15 Gew.-%, bevorzugt 0,1 Gew.-% bis 10 Gew.-%, besonders bevorzugt 0,2 Gew.-% bis 2,9 Gew.-% Mannitol,
jeweils bezogen auf die Gesamtmenge an a-D-Glucopyranosyl-1,1-D-Mannitol, α-D-Glucopyranosyl-1,6-D-Sorbitol, α-D-Glucopyranosyl-1,1-D-Sorbitol, Sorbitol und Mannitol
mit der Maßgabe,
dass das Gewichtsverhältnis von α-D-Glucopyranosyl-1,6-D-Sorbitol zu α-D-Glucopyranosyl-1,1-D-Mannitol größer als 53:47, insbesondere größer als 55:45 ist.

Besteht das erfindungsgemäße Süßungsmittel aus den oben genannten Substanzen, so addieren sich die angegebenen Gew.-% zu 100.

Zur Bestimmung der jeweiligen Gewichtsanteile können die Verfahren beschrieben in den ISOMALT Spezifikationen, ausgearbeitet im Rahmen der 69th JECFA (2008), veröffentlicht in den FAO JECFA Monographs 5 (2008), herangezogen werden.

Bevorzugt ist die Summe der Gew.-% von α-D-Glucopyranosyl-1,6-D-Sorbitol und α-D-Glucopyranosyl-1,1-D-Mannitol größer als 75, bevorzugt größer als 80, besonders bevorzugt größer als 86 bezogen auf das Gesamtgewicht der Trockensubstanz des Süßungsmittels.

Bevorzugt enthält das erfindungsgemäße Süßungsmittel weniger als 2,5 Gew.-%, insbesondere weniger als 0,3 Gew.-%, am meisten bevorzugt keine nachweisbaren Mengen an Saccharose, bezogen auf das Gesamtgewicht der Trockensubstanz des Süßungsmittels.

Einen weiteren Beitrag zur Lösung der vorstehend genannten Aufgaben leistet ein Verfahren zur Herstellung eines Süßungsmittels durch Umsetzung einer Kohlenhydratmischung enthaltend Isomaltulose, Saccharose und gegebenenfalls Trehalulose, Fruktose und Glukose und/oder weitere Polysaccharide mit Wasserstoff dadurch gekennzeichnet, dass die Umsetzung in Gegenwart mindestens eines Katalysators, der auf Ruthenium (Ru) und/oder mindestens einem Oxid des Rutheniums basiert, durchgeführt wird.
In diesem Verfahren wird bevorzugt durch Wasserstoff sowohl die Isomaltulose und gegebenenfalls Trehalulose zu 1,1-GPM und 1,6-GPS und gegebenenfalls zu 1,1-GPS katalytisch hydriert als auch Saccharose zu Fruktose und Glukose gespalten und diese zu Mannitol und Sorbitol hydriert. Die beiden Letztgenannten sind ebenfalls Zuckeraustauschstoffe und sind somit ideale Beiprodukte zu dem erhaltenen 1,1-GPM, 1,6-GPS und 1,1-GPS. Somit entspricht die Umsetzung in dem erfindungsgemäßen Verfahren einer katalytischen Hydrierung einhergehend mit einer Spaltung der Saccharose in Fruktose und Glukose.
Es ist daher bevorzugt, dass die Spaltung der Saccharose und die Hydrierung anderer, anwesender Kohlenhydrate simultan erfolgen.
In den erfindungsgemäßen Verfahren werden bevorzugt Katalysatoren eingesetzt, bei denen das Ruthenium (Ru) und/oder die rutheniumhaltige Verbindung auf einem Träger, insbesondere einem sauren oder kohlenstoffhaltigen Träger, immobilisiert vorliegen.

Die Umsetzung findet bevorzugt in einer wässrigen Lösung statt, so dass die Kohlenhydratmischung Wasser enthalten kann. Bevorzugt enthält die Kohlenhydratmischung somit 20 Gew.-% bis 80 Gew.-%, bevorzugt 30 Gew.-% bis 70 Gew.-%, besonders bevorzugt 40 Gew.-% bis 60 Gew.-% Wasser bezogen auf die gesamte Kohlenhydratmischung.
Der pH der wässrigen Lösung liegt bevorzugt im neutralen oder sauren Bereich entsprechend einem pH-Wert von kleiner 8.

Die im erfindungsgemäßen Verfahren eingesetzte Kohlenhydratmischung ist bevorzugt erhältlich durch die enzymatische Umsetzung von saccharosehaltigen, wässrigen Lösungen, wie beispielsweise wässrige Lösungen von Zucker aus Zuckerrüben oder Zuckerrohr, mit Isomaltulose Synthasen. Geeignete Isomaltulose Synthasen sind beispielsweise solche aus *Enterobacter sp.* strain FMB1, *Erwinia rhapontici, Klebsiella planticola* strain UQ14S, *Klebsiella pneumoniae* NK33-98-8, *Klebsiella sp.* LX3, *Pantoea dispersa* UQ68J, *Protaminobacter ruber* Z12, *Protaminobacter rubrum, Pseudomonas mesoacidophila* MX-45, *Serratia plymuthica.* Insbesondere Kohlenhydratmischungen erhältlich durch die enzymatische Umsetzung von saccharosehaltigen, wässrigen Lösungen mit Isomaltulose Synthasen aus *Protaminobacter rubrum,* insbesondere des Stammes *Protaminobacter rubrum* CBS 574.77, können vorteilhaft im erfindungsgemäßen Verfahren eingesetzt werden.
Bei der in der Kohlenhydratmischung enthaltenen Saccharose handelt es sich somit bevorzugt um Rest-Saccharose.
Bevorzugt enthält die in dem erfindungsgemäßen Verfahren eingesetzte Kohlenhydratmischung 0,01 Gew.-% bis 15 Gew.-%, bevorzugt 0,1 Gew.-% bis 5 Gew.-% und besonders bevorzugt 0,2 Gew.-% bis 2 Gew.-% Saccharose bezogen auf das Trockengewicht der gesamten Kohlenhydratmischung.

Bevorzugt enthält die in dem erfindungsgemäßen Verfahren eingesetzte Kohlenhydratmischung mindestens 70 Gew.-%, bevorzugt mindestens 80 Gew.-% und ganz bevorzugt mindestens 90 Gew.-% Isomaltulose bezogen auf das Trockengewicht der gesamten Kohlenhydratmischung.
Bevorzugt enthält die in dem erfindungsgemäßen Verfahren eingesetzte Kohlenhydratmischung 0,02 Gew.-% bis 30 Gew.-%, bevorzugt 0,1 Gew.-% bis 20 Gew.-%, besonders bevorzugt 0,2 Gew.-% bis 10 Gew.-% Trehalulose bezogen auf das Trockengewicht der gesamten Kohlenhydratmischung.

Die genannten Katalysatoren auf der Basis von Ruthenium (Ru) und/oder Rutheniumoxid haben sich im Hinblick auf einen vollständigen Umsatz der eingesetzten Edukte und auf eine äußerst hohe Selektivität bezüglich der vorgenannten Produkte überraschenderweise als anderen bekannten Hydrierkatalysatoren deutlich überlegen erwiesen.

Als Katalysatorträger kommen alle dem Fachmann geeignet erscheinende Feststoffe in Betracht. Solche sind beispielsweise Kohlenstoff in Form von zum Beispiel Aktivkohle, wie insbesondere auch saure Träger, wie beispielsweise Metalloxide, wie Al₂O₃, SiO₂, TeO₂, Mischoxide derselben oder auch MgO-SiO₂, ZrO₂-SiO₂ sowie Heteropolysäuren. Ferner sind zu nennen: Mineralsäuren, wie beispielsweise H₃PO₄ oder H₂SO₄, die auf festen, bevorzugt porösen, ebenso bevorzugt inerten Trägern aufgebracht sind, Kationentauscher, Salze von sauerstoffhaltigen Mineralsäuren, bevorzugt von Schwermetallen (Phosphate, Sulfate, Wolframate), Halogenide dreiwertiger Metalle (wie etwa AlCl₃) auf porösen Trägern, Zeolithe (H-Form) oder die sogenannten, mit H₂SO₄ behandelten Supersäuren ZrO₂ oder TiO₂.

Ferner sind Träger geeignet, die von ihrer Funktionalität her eher als neutral einzustufen sind, wie beispielsweise Aktivkohlen oder TiO2, die bevorzugt durch geeignete Imprägnierungsverfahren und/oder durch Aufbringung des Katalysatormetalls selber eine saure Funktionalität erhalten.

In diesem Zusammenhang ist es bevorzugt, dass diese Träger geeignete Porenvolumina aufweisen, die zur guten Anbindung und Aufnahme des Hydrierungskatalysators geeignet sind. Außerdem sind Gesamtporenvolumen nach DIN 66133 in einem Bereich von 0,01 bis 3 ml/g bevorzugt und in einem Bereich von 0,2 bis 1 ml/g besonders bevorzugt. Zudem ist es bevorzugt, dass die als Träger geeigneten Feststoffe eine Oberfläche in einem Bereich von 0,001 bis 1500 m²/g, vorzugsweise in einem Bereich von 10 bis 450 m²/g und darüber hinaus bevorzugt in einem Bereich von 10 bis 270 m²/g nach BET-Test gemäß DIN 66131 aufweisen. Als Träger für den Hydrierungskatalysator kann zum einen ein Schüttgut, das einen mittleren Teilchendurchmesser in einem Bereich von 0,1 bis 40 mm, vorzugsweise in einem Bereich von 0,8 bis 7 mm und darüber hinaus bevorzugt in einem Bereich von 1,5 bis 7 mm aufweist, eingesetzt werden. Ferner kann die Wand des Hydrierungsreaktors als inerter Träger dienen.
Als Aufbringtechniken des Hydrierungskatalysators sind insbesondere Eintauchen bzw. Imprägnieren oder das Einarbeiten in eine Trägermatrix zu nennen.

Es entspricht einer erfindungsgemäßen Ausführungsform, dass der saure Träger mindestens teilweise aus einer oxidischen Verbindung besteht. Derartige oxidische Verbindungen sollten mindestens eines der Elemente ausgewählt aus der Gruppe umfassend Si, Ti, Te, Zr, Al, P oder eine Kombination von mindestens zwei dieser Elemente aufweisen.

Bevorzugte saure Träger sind ausgewählt aus der Gruppe umfassend, bevorzugt bestehend aus Silicium-, Aluminium-, Tellur und Phosphoroxide, wobei Al₂O₃, SiO₂, TeO₂ und Mischoxide derselben besonders und Al₂O₃ ganz besonders bevorzugt sind.

In dem erfindungsgemäßen Verfahren können als Träger auch super saure Träger eingesetzt werden.
Diese sind dem Fachmann bekannt als solche Träger wie beispielsweise Zeolithe vom H-Y-Typ, bevorzugt mit einem Si-Al-Verhältnis >50, sowie saurer Ionentauscher mit entsprechender Temperaturbeständigkeit, wie etwa die unter dem Handelsnamen Amberlyst verfügbaren.

In einer alternativen Ausführungsform des erfindungsgemäßen Verfahren können als Träger auch neutrale Träger eingesetzt werden. Solche sind insbesondere ausgewählt aus der Liste bestehend aus elementarem Kohlenstoff, insbesondere Aktivkohlen, und TiO₂, wobei Aktivkohle besonders bevorzugt ist.

Das erfindungsgemäße Verfahren wird vorteilhaft bei erhöhten Temperaturen durchgeführt. Der bevorzugte Temperaturbereich beträgt 80 °C bis 150 °C, wobei als Verfahrenstemperatur die Temperatur betrachtet wird, die in der Kohlenhydratmischung, welche gegebenenfalls bereits erfindungsgemäßes Süßungsmittel enthält, gemessen wird.

Eine alternative Ausführungsform des erfindungsgemäßen Verfahren ist dadurch gekennzeichnet, dass das Verfahren bis zu einem Umsatz von 50 % bis 95 % bezogen auf die Hydrierung der Isomaltulose in einem Temperaturbereich zwischen 80 bis 120 °C und der weitere im wesentlichen 100 %ige Umsatz bezogen auf die Hydrierung der Isomaltulose in einem Temperaturbereich zwischen 100 °C bis 150 °C, bevorzugt 121 °C bis 150 °C, durchgeführt wird.
In diesem Zusammenhang ist es erfindungsgemäß bevorzugt, dass die beiden unterschiedlichen Temperaturbereiche räumlich voneinander getrennt sind, und in beiden Temperaturbereichen ein Katalysator, bei dem das Ruthenium (Ru) und/oder die rutheniumhaltige Verbindung auf einem oxidhaltigen Träger, wobei das Oxid insbesondere ausgewählt ist aus Al₂O₃ und TiO₂, immobilisiert vorliegen, eingesetzt wird.
In einer alternativen Ausführungsform ist es in diesem Zusammenhang erfindungsgemäß bevorzugt, dass die beiden unterschiedlichen Temperaturbereiche räumlich voneinander getrennt sind, in dem Temperaturbereich, der 80°C bis 120 °C beträgt, ein Katalysator, bei dem das Ruthenium (Ru) und/oder die rutheniumhaltige Verbindung auf einem oxidhaltigen Träger, wobei das Oxid insbesondere ausgewählt ist aus Al₂O₃ und TiO₂, immobilisiert vorliegen, eingesetzt wird, und in dem Temperaturbereich, der 100°C bis 150 °C, bevorzugt 121 °C bis 150 °C, beträgt, ein Katalysator, bei dem das Ruthenium (Ru) und/oder die rutheniumhaltige Verbindung auf einem kohlestoffhaltigen Träger immobilisiert vorliegen, eingesetzt wird.

Eine besondere Ausführungsform des erfindungsgemäßen Verfahrens ist dadurch gekennzeichnet, dass als Träger ein super saurer Träger eingesetzt wird und die Verfahrenstemperatur unter 120 °C, insbesondere 80 °C bis 110 °C beträgt.

Bezüglich des während der Hydrierungsreaktion eingestellten Druckes hat sich insbesondere ein Druck von wenigstens 15 bar, bevorzugt von wenigstens 30 bar, besonders bevorzugt wenigstens 40 bar, als vorteilhaft erwiesen. Besonders bevorzugt sind Werte zwischen 40 bar und 150 bar, insbesondere zwischen 40 bar und 90 bar, beispielsweise im Bereich von etwa 50 bis 60 bar.

Bevorzugt wird das Verfahren so lange durchgeführt, bis in dem erhaltenen Süßungsmittel keine Saccharose mehr nachweisbar ist.

Um das durch das erfindungsgemäße Verfahren erhaltene Süßungsmittel, das vorzugsweise als Kohlenhydratmischung in flüssiger Form vorliegt, in trockene Form zu überführen, kann das als Lösungsmittel vorliegende Wasser durch die Benutzung eines Verdampfers oder eines Trockners beispielsweise eines Fallfilmverdampfers oder eines Trommeltrockners oder eines Sprühtrockners entfernt werden.

Es kann vorteilhaft sein, das erhaltene Süßungsmittel mit weiteren Reinigungs- oder An- und/oder Abreicherungsschritten weiterzubehandeln.
So kann es vorteilhaft sein, den Gehalt an Mannitol durch einen weiteren Kristallisationschritt zu verringern, beispielsweise auf 0,02 Gew.-% bis 15 Gew.-%, bevorzugt auf 0,1 Gew.-% bis 10 Gew.-%, besonders bevorzugt auf 0,2 Gew.-% bis 2,9 Gew.-% bezogen auf die Trockenmasse des Süßungsmittels; dies ist wegen der geringen Wasserlöslichkeit des Mannitols gut möglich.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Ru-katalysierte Hydrierung einer Isomaltuloselösung enthaltend Saccharose bei 90 °C

Eine wässrige Lösung enthaltend 40 Gew.-% Isomaltulose und 3 Gew.-% Saccharose wird erfindungsgemäß an einem Ru-Katalysator, 1,5 Gew.-% auf Aluminiumoxid, bei 60bar Wasserstoff und 90°C über einem kontinuierlich betriebenen Festbettreaktor bei einer LHSV (Liquid Hourly Space Velocity) von 0, 47h⁻¹ hydriert. Die Apparatur bestand aus einem Rohrreaktor mit Luftbeheizung bzw. Luftkühlung mit einem Innendurchmesser des Reaktorrohres von 11 mm. Das Rohr war mit 19 ml Katalysator Noblyst ® 3001, Evonik Degussa GmbH, bestückt.
Der Wasserstoffvolumenstrom betrug 100 Nml/min.
Das Produkt zeigt ein Isomerenverhältnis 1,6-GPS zu 1,1-GPM von 56:44 bei einem Umsatz von 80% bezüglich der eingesetzten Isomaltulose und 24% bezüglich Saccharose. Dabei wurde die umgesetzte Isomaltulose mit einer Selektivität von nahezu 100 % zu Isomalt hydriert. Die Saccharose wird unter den Reaktionsbedingungen zu Mannitol und Sorbitol hydriert.

### Beispiel 2: Ru-katalysierte Hydrierung einer Isomaltuloselösung enthaltend Saccharose bei 120 °C

Wird dieselbe Einsatzlösung wie in Beispiel 1 abweichend bei 120°C unter ansonsten gleichen Bedingungen umgesetzt, so wird die eingesetzte Isomaltulose zu 95%, die Saccharose zu 93% hydriert. Auch hier wurde die umgesetzte Isomaltulose mit einer Selektivität von nahezu 100 % zu Isomalt hydriert. Das Isomerenverhältnis 1,6-GPS zu 1,1-GPM erreicht weiterhin 56:44. Die Saccharose wird unter den Reaktionsbedingungen zu Mannitol und Sorbitol hydriert.

### Beispiel 3: Ru-katalysierte (Ru/C) Hydrierung einer Isomaltuloselösung enthaltend Saccharose bei 90 °C

Eine wässrige Lösung enthaltend 40 Gew.-% Isomaltulose und 3 Gew.-% Saccharose wird erfindungsgemäß an einem Ru-Katalysator, 2 Gew.-% auf Aktivkohle, bei 60 bar Wasserstoff und 90°C über einem kontinuierlich betriebenen Festbettreaktor bei einer LHSV von 0,47h⁻¹ hydriert. Die Apparatur bestand aus einem Rohrreaktor mit Luftkühlung mit einem Innendurchmesser des Reaktorrohres von 11 mm. Das Rohr war mit 19 ml Katalysator Noblyst ® 3000, Evonik Degussa GmbH, bestückt.
Der Wasserstoffvolumenstrom betrug 100 Nml/min.
Das Produkt zeigt ein Isomerenverhältnis 1,6-GPS zu 1,1-GPM von 56:44 bei einem Umsatz von 96% bezüglich der eingesetzten Isomaltulose und 60% bezüglich Saccharose. Dabei wurde die umgesetzte Isomaltulose mit einer Selektivität von nahezu 100 % zu Isomalt hydriert. Die Saccharose wird unter den Reaktionsbedingungen zu Mannitol und Sorbitol hydriert.

### Beispiel 4: Ru-katalysierte (Ru/C) Hydrierung einer Isomaltuloselösung enthaltend Saccharose bei 120 °C

Wird dieselbe Einsatzlösung wie in Beispiel 3 abweichend bei 120°C unter ansonsten gleichen Bedingungen umgesetzt, so wird die eingesetzte Isomaltulose zu >99%, die Saccharose zu 98% hydriert. Auch hier wurde die umgesetzte Isomaltulose mit einer Selektivität von nahezu 100 % zu Isomalt hydriert. Das Isomerenverhältnis 1,6-GPS zu 1,1-GPM erreicht weiterhin 56:44. Die Saccharose wird unter den Reaktionsbedingungen zu Mannitol und Sorbitol hydriert.

### Beispiel 5: Ni-katalysierte, nicht erfindungsgemäße Hydrierung einer Isomaltuloselösung enthaltend Saccharose bei 90 °C

Eine wässrige Lösung enthaltend 40w% Isomaltulose und 3w% Saccharose wird mit 10,5 g eines Raney-Ni Katalysator, B 113 W, Evonik Degussa GmbH, bei 60 bar Wasserstoff und 90°C in einem Rührkesselreaktor hydriert. Die Apparatur bestand aus einem Parr RK2 Rührkesselreaktor mit Begasungsrührer, einem Nennvolumen von 1,8 L und einem Reaktionsvolumen von 1,2 L; die Hydrierung erfolgte isotherm ohne Korb im Slurry.
Nach 4 h wird ein vollständiger Umsatz der Isomaltulose zu 1,6-GPS und 1,1-GPM bei einem Isomerenverhältnis von 53:47 erhalten, ein Umsatz der Saccharose ist über diesen Zeitraum nicht zu erkennen. Dabei wurde die umgesetzte Isomaltulose mit einer Selektivität von nahezu 100 % zu Isomalt hydriert.

## Patentansprüche

1. Süßungsmittel enthaltend
20 Gew.-% bis 75 Gew.-% α-D-Glucopyranosyl-1,6-D-Sorbitol,
20 Gew.-% bis 75 Gew.-% α-D-Glucopyranosyl-1,1-D-Mannitol,
0,02 Gew.-% bis 15 Gew.-% α-D-Glucopyranosyl-1,1-D-Sorbitol,
0,02 Gew.-% bis 15 Gew.-% Sorbitol und
0,02 Gew.-% bis 15 Gew.-% Mannitol,
jeweils bezogen auf die Gesamtmenge an α-D-Glucopyranosyl-1,1-D-Mannitol, α-D-Glucopyranosyl-1,6-D-Sorbitol, α-D-Glucopyranosyl-1,1-D-Sorbitol, Sorbitol und Mannitol
mit der Maßgabe,
dass das Gewichtsverhältnis von α-D-Glucopyranosyl-1,6-D-Sorbitol zu α-D-Glucopyranosyl-1,1-D-Mannitol größer als 53:47 ist.

2. Süßungsmittel nach Anspruch 1, **dadurch gekennzeichnet, dass** die Summe der Gew.-% von α-D-Glucopyranosyl-1,6-D-Sorbitol und α-D-Glucopyranosyl-1,1-D-Mannitol größer als 86 bezogen auf das Gesamtgewicht der Trockensubstanz des Süßungsmittels ist.

3. Verfahren zur Herstellung eines Süßungsmittels durch Umsetzung einer Kohlenhydratmischung enthaltend Isomaltulose und Saccharose mit Wasserstoff, **dadurch gekennzeichnet, dass** die Umsetzung in Gegenwart mindestens eines Katalysators, der auf Ruthenium (Ru) und/oder mindestens einem Oxid des Rutheniums basiert, durchgeführt wird.

4. Verfahren gemäß Anspruch 3, **dadurch gekennzeichnet, dass** die Kohlenhydratmischung 0,01 Gew.-% bis 15 Gew.-% Saccharose bezogen auf das Trockengewicht der gesamten Kohlenhydratmischung enthält.

5. Verfahren gemäß Anspruch 3 oder 4, **dadurch gekennzeichnet, dass** die Kohlenhydratmischung 0,02 Gew.-% bis 30 Gew.-% Trehalulose bezogen auf das Trockengewicht der gesamten Kohlenhydratmischung enthält.

6. Verfahren gemäß mindestens einem der Ansprüche 3 bis 5, **dadurch gekennzeichnet, dass** die Kohlenhydratmischung 20 Gew.-% bis 70 Gew.-% Wasser bezogen auf die gesamte Kohlenhydratmischung enthält.

7. Verfahren gemäß mindestens einem der Ansprüche 3 bis 6, **dadurch gekennzeichnet, dass** bei dem Katalysator das Ruthenium (Ru) und/oder die rutheniumhaltige Verbindung auf einem Träger immobilisiert vorliegen.

8. Verfahren gemäß Anspruch 7, **dadurch gekennzeichnet, dass** der Träger ein Gesamtporenvolumen nach DIN 66133 in einem Bereich von 0,01 bis 3 ml/g aufweist.

9. Verfahren gemäß Anspruch 7 oder 8, **dadurch gekennzeichnet, dass** der Träger eine Oberfläche in einem Bereich von 0,001 bis 1500 m²/g nach BET-Test gemäß DIN 66131 aufweist.

10. Verfahren gemäß mindestens einem der Ansprüche 7 bis 9, **dadurch gekennzeichnet, dass** der Träger ein neutraler Träger, insbesondere TiO₂ oder Aktivkohle, ist.

11. Verfahren gemäß mindestens einem der Ansprüche 7 bis 9, **dadurch gekennzeichnet, dass** der Träger ausgewählt ist aus der Gruppe umfassend saure Oxide und Mischoxide, natürliche und synthetische silikatische Stoffe.

12. Verfahren gemäß mindestens einem der Ansprüche 7 bis 9, **dadurch gekennzeichnet, dass** der Träger mindestens teilweise aus einer oxidischen Verbindung mindestens eines der Elemente ausgewählt aus der Gruppe umfassend Si, Ti, Te, Zr, Al, P oder eine Kombination von mindestens zwei dieser Elemente besteht, insbesondere aus Al₂O₃.

13. Verfahren gemäß mindestens einem der Ansprüche 7 bis 9, **dadurch gekennzeichnet, dass** der Träger ein super saurer Träger ausgewählt aus Zeolithen vom H-Y-Typ oder aus sauren Ionentauschern ist.

14. Verfahren gemäß mindestens einem der Ansprüche 3 bis 12, **dadurch gekennzeichnet, dass** das Verfahren in einem Temperaturbereich durchgeführt wird, der 80°C bis 150 °C beträgt.

15. Verfahren gemäß Anspruch 14, **dadurch gekennzeichnet, dass** das Verfahren bei einer Temperatur kleiner 120 °C durchgeführt wird.

16. Verfahren gemäß mindestens einem der Ansprüche 3 bis 14, **dadurch gekennzeichnet, dass** das Verfahren bis zu einem Umsatz von 50 % bis 95 % bezogen auf die Hydrierung der Isomaltulose in einem Temperaturbereich zwischen 80 bis 120 °C und der weitere im wesentlichen 100 %ige Umsatz bezogen auf die Hydrierung der Isomaltulose in einem Temperaturbereich zwischen 100 °C bis 150 °C durchgeführt wird.

17. Verfahren gemäß Anspruch 16, **dadurch gekennzeichnet, dass** die beiden unterschiedlichen Temperaturbereiche räumlich voneinander getrennt sind, und in beiden Temperaturbereichen ein Katalysator, bei dem das Ruthenium (Ru) und/oder die rutheniumhaltige Verbindung auf einem oxidhaltigen Träger, wobei das Oxid insbesondere ausgewählt ist aus Al₂O₃ und TiO₂, immobilisiert vorliegen, eingesetzt wird.

18. Verfahren gemäß Anspruch 16, **dadurch gekennzeichnet, dass** die beiden unterschiedlichen Temperaturbereiche räumlich voneinander getrennt sind, in dem Temperaturbereich, der 80°C bis 150 °C beträgt, ein Katalysator, bei dem das Ruthenium (Ru) und/oder die rutheniumhaltige Verbindung auf einem oxidhaltigen Träger, wobei das Oxid insbesondere ausgewählt ist aus Al₂O₃ und TiO₂, immobilisiert vorliegen, eingesetzt wird, und in dem Temperaturbereich, der 100°C bis 150 °C beträgt, ein Katalysator, bei dem das Ruthenium (Ru) und/oder die rutheniumhaltige Verbindung auf einem kohlestoffhaltigen Träger immobilisiert vorliegen, eingesetzt wird.

19. Verfahren gemäß mindestens einem der Ansprüche 3 bis 18, **dadurch gekennzeichnet, dass** der eingestellte Druck während des Verfahrens mindestens 15 bar beträgt.

## Claims

1. A sweetener containing
20 wt.% to 75 wt.% of α-D-glucopyranosyl-1,6-D-sorbitol,
20 wt.% to 75 wt.% of α-D-glucopyranosyl-1,1-D-mannitol,
0.02 wt.% to 15 wt.% of α-D-glucopyranosyl-1,1-D-sorbitol,
0.02 wt.% to 15 wt.% of sorbitol and
0.02 wt.% to 15 wt.% of mannitol,
in each case relative to the total amount of α-D-glucopyranosyl-1,1-D-mannitol, α-D-glucopyranosyl-1,6-D-sorbitol, α**-**D-glucopyranosyl-1,1-D-sorbitol, sorbitol and mannitol
with the proviso,
that the weight ratio of α-D-glucopyranosyl-1,6-D-sorbitol to α-D-glucopyranosyl-1,1-D-mannitol is greater than 53:47.

2. A sweetener according to claim 1, **characterized in that** the sum of the wt.% of α-D-glucopyranosyl-1,6-D-sorbitol and α-D-glucopyranosyl-1,1-D-mannitol is greater than 86 relative to the total weight of dry matter of the sweetener.

3. A method of production of a sweetener by reaction of a carbohydrate mixture containing isomaltulose and sucrose with hydrogen, **characterized in that** the reaction is carried out in the presence of at least one catalyst, which is based on ruthenium (Ru) and/or at least one oxide of ruthenium.

4. A method according to claim 3, **characterized in that** the carbohydrate mixture contains 0.01 wt.% to 15 wt.% of sucrose relative to the dry weight of the total carbohydrate mixture.

5. A method according to claim 3 or 4, **characterized in that** the carbohydrate mixture contains 0.02 wt.% to 30 wt.% of trehalulose relative to the dry weight of the total carbohydrate mixture.

6. A method according to at least one of claims 3 to 5, **characterized in that** the carbohydrate mixture contains 20 wt.% to 70 wt.% of water relative to the total carbohydrate mixture.

7. A method according to at least one of claims 3 to 6, **characterized in that** in the catalyst, the ruthenium (Ru) and/or the ruthenium-containing compound is immobilized on a support.

8. A method according to claim 7, **characterized in that** the support has a total pore volume according to DIN 66133 in a range from 0.01 to 3 ml/g.

9. A method according to claim 7 or 8, **characterized in that** the support has a surface area in a range from 0.001 to 1500 m²/g in the BET test according to DIN 66131.

10. A method according to at least one of claims 7 to 9, **characterized in that** the support is a neutral support, in particular TiO₂ or activated charcoal.

11. A method according to at least one of claims 7 to 9, **characterized in that** the support is selected from the group comprising acid oxides and mixed oxides, natural and synthetic silicates.

12. A method according to at least one of claims 7 to 9, **characterized in that** the support consists at least partially of an oxide compound of at least one of the elements selected from the group comprising Si, Ti, Te, Zr, Al, P or a combination of at least two of these elements, in particular of Al₂O₃.

13. A method according to at least one of claims 7 to 9, **characterized in that** the support is a super-acid support selected from zeolites of the H-Y type or from acid ion exchangers.

14. A method according to at least one of claims 3 to 12, **characterized in that** the method is carried out in a temperature range from 80°C to 150°C.

15. A method according to claim 14, **characterized in that** the method is carried out at a temperature below 120°C.

16. A method according to at least one of claims 3 to 14, **characterized in that** the method is carried out up to a conversion of 50% to 95% relative to the hydrogenation of the isomaltulose in a temperature range between 80 to 120°C and the further, essentially 100% conversion relative to the hydrogenation of the isomaltulose in a temperature range between 100°C to 150°C.

17. A method according to claim 16, **characterized in that** the two different temperature ranges are spatially separate from one another, and in both temperature ranges a catalyst is used in which ruthenium (Ru) and/or the ruthenium-containing compound is immobilized on an oxide-containing support, the oxide being selected in particular from Al₂O₃ and TiO₂.

18. A method according to claim 16, **characterized in that** the two different temperature ranges are spatially separate from one another, and in the temperature range from 80°C to 150°C, a catalyst is used in which ruthenium (Ru) and/or the ruthenium-containing compound is immobilized on an oxide-containing support, the oxide being selected in particular from Al₂O₃ and TiO₂, and in the temperature range from 100°C to 150°C, a catalyst is used in which ruthenium (Ru) and/or the ruthenium-containing compound is immobilized on a carbon-containing support.

19. A method according to at least one of claims 3 to 18, **characterized in that** the pressure used during the process is at least 15 bar.

## Revendications

1. Édulcorant contenant
20 % en poids à 75 % en poids d'α-D-glucopyranosyl-1,6-D-sorbitol,
20 % en poids à 75 % en poids d'α-D-glucopyranosyl-1,1-D-mannitol,
0,02 % en poids à 15 % en poids d'α-D-glucopyranosyl-1,1-D-sorbitol,
0,02 % en poids à 15 % en poids de sorbitol et
0,02 % en poids à 15 % en poids de mannitol,
chaque fois par rapport à la quantité totale d'α-D-glucopyranosyl-1,1-D-mannitol, α-D-glucopyranosyl-1,6-D-sorbitol, α-D-glucopyranosyl-1,1-D-sorbitol, sorbitol et mannitol
étant entendu
que le rapport pondéral de l'α-D-glucopyranosyl-1,6-D-sorbitol à l'α-D-glucopyranosyl-1,1-D-mannitol est supérieur à 53:47.

2. Édulcorant selon la revendication 1, **caractérisé en ce que** la somme des % en poids d'α-D-glucopyranosyl-1,6-D-sorbitol et d'α-D-glucopyranosyl-1,1-D-mannitol est supérieure à 86 par rapport au poids total de la matière sèche de l'édulcorant.

3. Procédé pour la préparation d'un édulcorant par mis en réaction d'un mélange de glucides contenant de l'isomaltulose et du saccharose avec de l'hydrogène, **caractérisé en ce qu'**on effectue la réaction en présence d'au moins un catalyseur qui est à base de ruthénium (Ru) et/ou d'au moins un oxyde du ruthénium.

4. Procédé selon la revendication 3, **caractérisé en ce que** le mélange de glucides contient 0,01 % en poids à 15 % en poids de saccharose par rapport au poids sec du mélange total de glucides.

5. Procédé selon la revendication 3 ou 4, **caractérisé en ce que** le mélange de glucides contient 0,02 % en poids à 30 % en poids de tréhalulose par rapport au poids sec du mélange total de glucides.

6. Procédé selon au moins l'une quelconque des revendications 3 à 5, **caractérisé en ce que** le mélange de glucides contient 20 % en poids à 70 % en poids d'eau par rapport au mélange total de glucides.

7. Procédé selon au moins l'une quelconque des revendications 3 à 6, **caractérisé en ce que** dans le catalyseur le ruthénium (Ru) et/ou le composé contenant du ruthénium se trouve(nt) immobilisé(s) sur un support.

8. Procédé selon la revendication 7, **caractérisé en ce que** le support présente un volume total de pores selon DIN 66133 dans une plage de 0,01 à 3 ml/g.

9. Procédé selon la revendication 7 ou 8, **caractérisé en ce que** le support présente une surface dans une plage de 0,001 à 1 500 m²/g conformément au test BET selon DIN 66131.

10. Procédé selon au moins l'une quelconque des revendications 7 à 9, **caractérisé en ce que** le support est un support neutre, en particulier TiO₂ ou du charbon actif.

11. Procédé selon au moins l'une quelconque des revendications 7 à 9, **caractérisé en ce que** le support est choisi dans le groupe comprenant des oxydes acides et des oxydes mixtes, des matières de type silicate naturelles et synthétiques.

12. Procédé selon au moins l'une quelconque des revendications 7 à 9, **caractérisé en ce que** le support consiste au moins en partie en un composé de type oxyde d'au moins l'un des éléments choisis dans le groupe comprenant Si, Ti, Te, Zr, Al, P ou une association d'au moins deux de ces éléments, en particulier en Al₂O₃.

13. Procédé selon au moins l'une quelconque des revendications 7 à 9, **caractérisé en ce que** le support est un support super-acide choisi parmi les zéolithes du type H-Y ou les échangeurs d'ions acides.

14. Procédé selon au moins l'une quelconque des revendications 3 à 12, **caractérisé en ce que** le procédé est mis en oeuvre dans une plage de température qui va de 80 °C à 150 °C.

15. Procédé selon la revendication 14, **caractérisé en ce que** le procédé est mis en oeuvre à une température inférieure à 120 °C.

16. Procédé selon au moins l'une quelconque des revendications 3 à 14, **caractérisé en ce que** le procédé est effectué jusqu'à un taux de conversion de 50 % à 95 %, sur la base de l'hydrogénation de l'isomaltulose, dans une zone de température comprise entre 80 et 120 °C et jusqu'à la conversion plus poussée pratiquement à 100 %, sur la base de l'hydrogénation de l'isomaltulose, dans une zone de température comprise entre 100 °C et 150 °C.

17. Procédé selon la revendication 16 **caractérisé en ce que** les deux différentes zones de températures différentes sont séparées spatialement l'une de l'autre, et dans les deux zones de températures on utilise un catalyseur dans lequel le ruthénium (Ru) et/ou le composé contenant du ruthénium se trouve(nt) immobilisé(s) sur un support de type oxyde, l'oxyde étant en particulier choisi parmi Al₂O₃ et TiO₂.

18. Procédé selon la revendication 16 **caractérisé en ce que** les deux zones de températures différentes sont séparées spatialement l'une de l'autre, dans la zone de température qui vaut de 80 °C à 150 °C on utilise un catalyseur dans lequel le ruthénium (Ru) et/ou le composé contenant du ruthénium se trouve(nt) immobilisé(s) sur un support de type oxyde, l'oxyde étant en particulier choisi parmi Al₂O₃ et TiO₂, et dans la zone de température qui vaut de 100 °C à 150 °C on utilise un catalyseur dans lequel le ruthénium (Ru) et/ou le composé contenant du ruthénium se trouve(nt) immobilisé(s) sur un support contenant du charbon.

19. Procédé selon au moins l'une quelconque des revendications 3 à 18, **caractérisé en ce que** la pression ajustée pendant le processus est d'au moins 15 bars.
